# EUROPEAN PATENT APPLICATION

(11) **EP 4 682 527 A1**
(43) Date of publication of application: **21.01.2026**
(21) Application number: 23927676.9
(22) Date of filing: 02.05.2023
(51) Int. Cl.: G01N 27/18, G01N 25/18, G01K 13/024

(54) **GAS SENSING ELEMENT**

(30) Priority: 16.03.2023 KR 20230034808
(71) Applicant: Inner Sensor Co., Ltd., Pohang-si, Gyeongsangbuk-do 37668 (KR)
(72) Inventor: KANG, Moon Sik, Pohang-si, Gyeongsangbuk-do 37666 (KR)
(74) Representative: Casalonga
(86) International application number: PCT/KR2023/005994
(87) International publication number: WO 2024/190955

(57) **Abstract**

A gas sensing element comprises: a substrate having mutually spaced reference region and sensing region, with an inlet located in the reference region through which gas can enter the interior of the substrate, an outlet located in the sensing region through which gas can be discharged from the interior of the substrate, and a connecting channel formed inside that connects the inlet and outlet; a heater disposed adjacent to the outlet and configured to heat a second gas existing around the outlet to generate upward airflow, thereby naturally convecting a first gas existing around the inlet through the inlet, the connecting channel, and the outlet; and first and second temperature sensors respectively disposed around the inlet and outlet and configured to sense temperature changes of the first and second gases. Thus, the gas sensing element can secure excellent sensitivity.

## Description

### BACKGROUND OF THE INVENTION

### 1. Field of the Invention

Embodiments of the present invention relate to a gas sensing element. More specifically, embodiments of the present invention relate to a gas sensing element that detects the presence of gas or measures gas concentration by sensing resistance changes due to heat transferred through thermal conduction.

### 2. Description of the Related Art

Hydrogen gas detection technologies are broadly classified into thermal semiconductor type, catalytic combustion type, and thermal conductivity type. Currently, technologies under research and development include optical type, FET (Field Effect Transistor) type, and composite material permeable thin film type, etc.

The thermal semiconductor type measures electrical resistance changes caused by gas adsorption on the surface of metal oxide semiconductors as resistance value changes appearing across metal wiring terminals. The catalytic combustion type consists of two elements: a detection specimen that reacts to combustible gas and a compensation specimen that does not react, measuring the temperature rise of the detection specimen when exposed to combustible gas through resistance differences with the compensation specimen. The thermal conductivity type measures temperature changes in a heating element due to differences in gas thermal conductivity.

These methods can be selected according to hydrogen concentration. For example, thermal semiconductor type is used for low-concentration hydrogen detection, while catalytic combustion type is used for high-concentration hydrogen detection.

Catalytic combustion type hydrogen sensors have the advantage of detecting high concentrations of hydrogen, but have problems with long-term reliability due to catalyst deterioration.

As an alternative, thermal conductivity type hydrogen sensors that can be used for high-concentration measurement have been proposed. Since the conductivity of gas or water vapor is a physical property, catalyst quality degradation or poisoning does not occur, enabling stable conditions over long periods.

Meanwhile, thermal conductivity type gas sensing elements include a reference specimen and a detection specimen. The detection specimen and reference specimen are respectively mounted in a first package having an open cap and a second package having a closed cap. Furthermore, heaters for heating gas temperature are provided inside each of the first and second packages.

However, thermal conductivity type gas sensing elements use a structure that forms sensor electrodes serving as both heaters and temperature sensors in two separate packages, generating temperature changes and deviations in each element when hydrogen gas enters, resulting in high unit costs and significant manufacturing costs.

Furthermore, in existing thermal conductivity type gas sensing elements, gas reaches the sensing electrodes located on the sensor surface through surface laminar flow air movement rather than effective convection that conducts heat by reaching the sensing electrodes. The surface laminar flow method has low air flow efficiency, resulting in poor sensitivity of the gas sensing element.

### SUMMARY OF THE INVENTION

Embodiments of the present invention provide a gas sensing element capable of achieving excellent sensitivity by having improved air flow efficiency through convection.

According to some embodiments of the present invention, a gas sensing element comprises: a substrate having mutually spaced reference region and sensing region, with an inlet located in the reference region through which gas can enter the interior of the substrate, an outlet located in the sensing region through which gas can be discharged from the interior of the substrate, and a connecting channel formed inside that connects the inlet and outlet; a heater disposed adjacent to the outlet and configured to heat a second gas existing around the outlet to generate upward airflow, thereby naturally convecting a first gas existing around the inlet through the inlet, the connecting channel, and the outlet; and first and second temperature sensors respectively disposed around the inlet and outlet and configured to sense temperatures of the first and second gases.

In an example embodiment of the present invention, each of the first or second temperature sensors may be configured to surround the inlet or outlet.

In an example embodiment of the present invention, the heater may be formed of at least one metal selected from Pt, Pt/Ti, Pt/Ta, Ir, Mo, and W.

In an example embodiment of the present invention, a heat transfer member configured to surround the inlet or outlet may be further provided.

Here, the heat transfer member may include a metal ring surrounding the outlet and an insulating layer pattern embedded within the metal ring.

Here, the second temperature sensor may be configured to surround the heat transfer member around the outlet, and an insulating member may be additionally provided to surround the second temperature sensor.

In an example embodiment of the present invention, the inlet may be arranged radially in plurality around the center of the reference region, and the outlet may be arranged radially in plurality around the center of the sensing region.

Here, the first temperature sensor may extend in a zigzag manner along the boundaries of the inlets, and the second temperature sensor may extend in a zigzag manner along the boundaries of the outlets.

In an example embodiment of the present invention, the reference region may be formed to surround the sensing region, the inlet may be arranged radially in plurality around the center of the sensing region, and the outlet may be arranged radially in plurality around the center of the sensing region.

Here, the first temperature sensor may extend along the inlets, and the second temperature sensor may extend in a zigzag manner along the boundaries of the outlets.

According to example embodiments, the gas sensing element includes a heater selectively disposed adjacent to the outlet. The heater heats the second gas existing around the outlet, reducing gas density, which may generate upward airflow around the outlet, and the heater disposed adjacent to the outlet functions as a thermal convection pump. Therefore, the gas sensing element can secure excellent sensitivity by facilitating gas flow using natural convection phenomena instead of conventional surface laminar flow.

### BRIEF DESCRIPTION OF THE DRAWINGS

The above and other features and advantages of the present invention will become more apparent by describing in detailed example embodiments thereof with reference to the accompanying drawings, in which:
FIG. 1 is a cross-sectional view illustrating a gas sensing element according to an example embodiment of the present invention;
FIG. 2 is a perspective view for explaining the gas sensing element of FIG. 1;
FIG. 3 is a plan view illustrating another example of the sensing region of FIG. 1;
FIG. 4 is a plan view illustrating a gas sensing element according to an embodiment of the present invention; and
FIG. 5 is a plan view illustrating a gas sensing element according to an embodiment of the present invention.

### DESCRIPTION OF THE EMBODIMENTS

The invention is described more fully hereinafter with reference to the accompanying drawings, in which embodiments of the invention are shown. This invention may, however, be embodied in many different forms and should not be construed as limited to the embodiments set forth herein. Rather, these embodiments are provided so that this disclosure will be thorough and complete, and will fully convey the scope of the invention to those skilled in the art. In the drawings, the sizes and relative sizes of layers and regions may be exaggerated for clarity.

It will be understood that when an element or layer is referred to as being "on", "connected to" or "coupled to" another element or layer, it can be directly on, connected or coupled to the other element or layer or intervening elements or layers may be present. In contrast, when an element is referred to as being "directly on," "directly connected to" or "directly coupled to" another element or layer, there are no intervening elements or layers present. Like numbers refer to like elements throughout. As used herein, the term "and/or" includes any and all combinations of one or more of the associated listed items.

It will be understood that, although the terms first, second, third etc. may be used herein to describe various elements, components, regions, layers and/or sections, these elements, components, regions, layers and/or sections should not be limited by these terms. These terms are only used to distinguish one element, component, region, layer or section from another region, layer or section. Thus, a first element, component, region, layer or section discussed below could be termed a second element, component, region, layer or section without departing from the teachings of the present invention.

Spatially relative terms, such as "beneath", "below", "lower", "above", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if the device in the figures is turned over, elements described as "below" or "beneath" other elements or features would then be oriented "above" the other elements or features. Thus, the exemplary term "below" can encompass both an orientation of above and below. The device may be otherwise oriented (rotated 90 degrees or at other orientations) and the spatially relative descriptors used herein interpreted accordingly.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

Embodiments of the invention are described herein with reference to cross-section illustrations that are schematic illustrations of idealized embodiments (and intermediate structures) of the invention. As such, variations from the shapes of the illustrations as a result, for example, of manufacturing techniques and/or tolerances, are to be expected. Thus, embodiments of the invention should not be construed as limited to the particular shapes of regions illustrated herein but are to include deviations in shapes that result, for example, from manufacturing. For example, an implanted region illustrated as a rectangle will, typically, have rounded or curved features and/or a gradient of implant concentration at its edges rather than a binary change from implanted to non-implanted region. Likewise, a buried region formed by implantation may result in some implantation in the region between the buried region and the surface through which the implantation takes place. Thus, the regions illustrated in the figures are schematic in nature and their shapes are not intended to illustrate the actual shape of a region of a device and are not intended to limit the scope of the invention.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

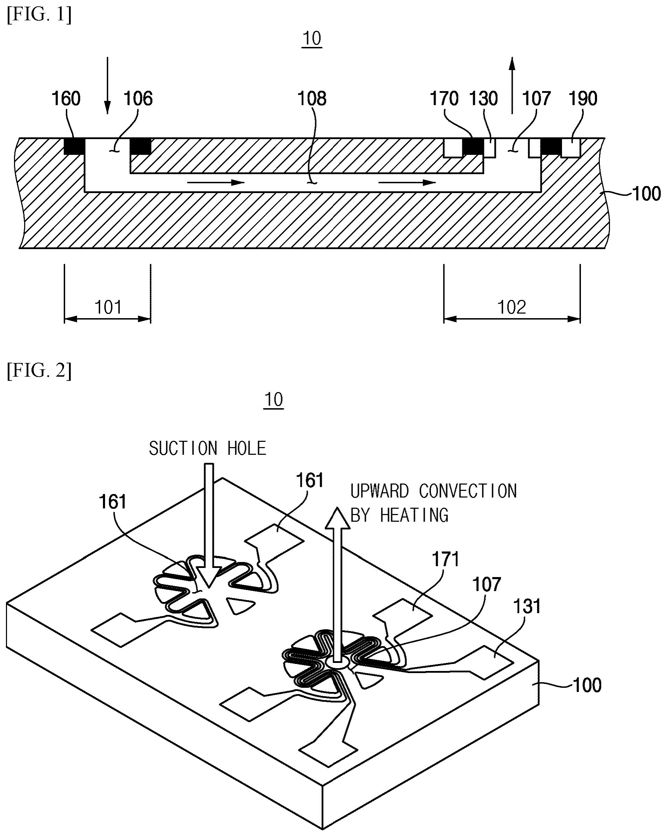

FIG. 1 is a cross-sectional view illustrating a gas sensing element according to an example embodiment of the present invention. FIG. 2 is a perspective view for explaining the gas sensing element of FIG. 1.

Referring to FIGS. 1 and 2, a gas sensing element 10 according to an example embodiment of the present invention includes a substrate 100, a heater 130, a first temperature sensor 160, and a second temperature sensor 170.

The substrate 100 may include a semiconductor substrate such as a silicon substrate or zirconium substrate.

The substrate 100 includes a reference region 101 and a sensing region 102. The reference region 101 and the sensing region 102 are defined to be spaced apart from each other along the horizontal direction.

The reference region 101 corresponds to a region that enables securing reference data regarding temperature by sensing the temperature of adjacent gas. Meanwhile, the sensing region 102 corresponds to a region that enables securing sensing data regarding temperature by sensing the temperature of adjacent gas. By securing a temperature difference value between the reference data and sensing data, the presence of gas, particularly hydrogen gas, and further the concentration of hydrogen gas can be measured.

For example, hydrogen gas can have thermal conductivity up to 10 times greater than other types of gases. Thus, when hydrogen gas concentration increases, heat generated from the adjacent heater 130 is lost to the surroundings, causing relatively large heat loss. Therefore, sensing data due to heat loss may change the temperature difference value with existing reference data. Through this, the presence and concentration of hydrogen gas can be measured.

For example, when hydrogen gas is not present around the sensing region, the temperature difference value can be maintained within a specific range. Conversely, when hydrogen gas is present around the sensing region, the temperature difference value deviates from the specific range. The concentration of hydrogen gas can be detected according to the change amount of the temperature difference value.

An inlet 106, outlet 107, and connecting channel 108 are formed in the substrate 100.

The inlet 106 is located in the reference region 101 and extends along the vertical direction. The inlet 106 provides a flow path for gas to enter the interior of the substrate 100.

Meanwhile, the outlet 107 is located in the sensing region 102 and extends along the vertical direction. The outlet 107 provides a flow path for discharging gas that entered from the interior of the substrate. Additionally, a connecting channel 108 that connects the inlet 106 with outlet 107 is formed inside the substrate. Therefore, gas can enter through the inlet 106, pass through the connecting channel 108, and be discharged through the outlet 107.

The heater 130 is selectively disposed adjacent to the outlet 107. That is, the heater 130 is disposed only at the outlet 107 and is not disposed around the inlet 106. That is, when the heater 130 operates, gas adjacent to the outlet 107 radiates heat generated from the heater 130. This not only relatively reduces the temperature of the heater 130 but also cools the gas adjacent to the outlet 107.

The heater 130 includes heating electrodes 131 connected to both ends thereof. The heating electrodes 131 can apply power to the heater.

Particularly, when the gas contains hydrogen, the gas adjacent to the outlet 107 has a relatively greater temperature decrease than when hydrogen is absent due to hydrogen gas having relatively large thermal conductivity.

The heater 130 is configured to heat gas existing around the outlet to generate upward airflow, thereby naturally convecting gas existing around the inlet 106 through the inlet 106, connecting channel 108, and outlet 107.

That is, the heater 130 heats the second gas existing around the outlet 107, reducing gas density. This creates upward airflow around the outlet 107. The heater 130 disposed adjacent to the outlet 10 can function as a thermal convection pump.

Meanwhile, the first gas adjacent to the inlet 106 where the heater 130 is not disposed has relatively greater density compared to the heated second gas. This creates downward airflow around the inlet 106. Meanwhile, the connecting channel 108 that connects the inlet 106 and outlet 107 is provided. Therefore, as the heater 130 that can function as a thermal convection pump operates, gas naturally flows through the inlet 106, connecting channel 108, and outlet 107, creating natural convection phenomena.

Consequently, natural convection phenomena occur instead of conventional surface laminar flow, enabling the gas sensing element 10 to secure excellent sensitivity.

The first temperature sensor 160 is disposed around the inlet 106. The first temperature sensor 160 senses temperature changes of the first gas around the inlet 106. At this time, temperature data sensed by the first temperature sensor 160 can be used as reference data.

The first temperature sensor 160 includes first sensing electrodes 161 connected to both ends. The first sensing electrodes 161 can apply power to the temperature sensor itself. The first temperature sensor 160 can sense the temperature of the first gas by detecting resistance value changes in current flowing through the applied power.

The second temperature sensor 170 is disposed around the outlet 107. The second temperature sensor 170 senses temperature changes of the second gas around the outlet 107. At this time, temperature data sensed by the second temperature sensor 170 can be used as sensing data.

The second temperature sensor 170 includes second sensing electrodes 171 connected to both ends. The second sensing electrodes 171 can apply power to the temperature sensor itself. The second temperature sensor 170 can sense the temperature of the second gas by detecting resistance value changes in current flowing through the applied power.

Regardless of the presence of hydrogen gas, when the heater 130 operates, gas flow occurs smoothly through the inlet 106, connecting passage 108, and outlet 107 due to natural convection phenomena. At this time, the first temperature sensor 160 and second temperature sensor 170 can sense the temperatures of the first and second gases, respectively.

If the second gas does not contain hydrogen, heat generated from the heater 130 is transferred to the second gas, and the second gas can have a relatively high temperature. Conversely, if the second gas contains hydrogen, heat generated from the heater 130 is transferred to the second gas. As hydrogen gas contained in the second gas has relatively large thermal conductivity and easily radiates heat externally, the second gas can have a relatively lower temperature compared to when there is no hydrogen gas.

That is, as the second gas rapidly radiates heat generated from the heater 130 externally, the gas sensing element 10 can have excellent response speed. Furthermore, the gas sensing element 10 can secure superior sensitivity characteristics compared to other methods even for relatively small amounts of gas concentration changes.

In an example embodiment of the present invention, the first and second temperature sensors 160, 170 may be configured to surround the inlet 106 or outlet 107. This enables more rapid and accurate sensing of temperatures of the first air and second air adjacent to the inlet 106 or outlet 107.

In one embodiment of the present invention, the heater 130 may use metals such as Pt, Pt/Ti, Pt/Ta, Ir, Mo, W, etc.

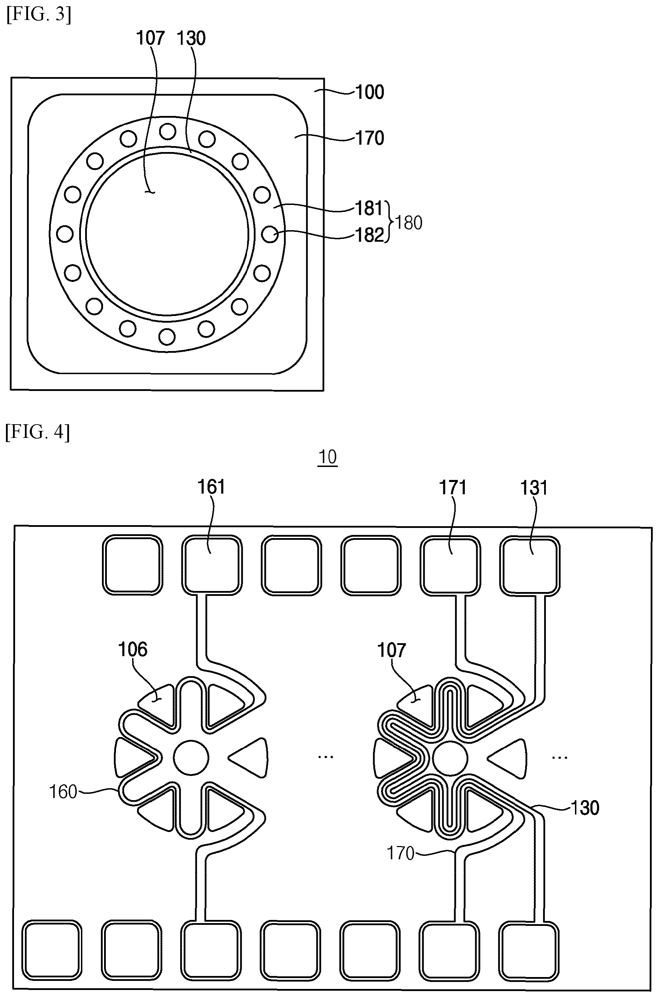

FIG. 3 is a plan view illustrating another example of the sensing region of FIG. 1.

Referring to FIGS. 1 through 3, a heat transfer member 180 configured to surround the outlet 107 may be additionally provided. At this time, the heat transfer member 180 may be interposed between the heater 130 and second temperature sensor 170.

Here, the heat transfer member 180 may include a metal ring 181 surrounding the outlet 107 and an insulating layer pattern 182 embedded within the metal ring.

The heat transfer member 180 can effectively transfer heat of air heated by heat generated from the heater 130 to the second temperature sensor 170.

The metal ring 181 may be made of metal materials having excellent thermal conductivity such as gold, silver, aluminum, copper, etc.

The insulating layer pattern 182 is embedded within the metal ring 181. The upper surface of the insulating layer pattern 182 is partially exposed. Therefore, the thermal conductivity of the heat transfer member 180 can be maintained constant even in humid environments with high humidity. The insulating layer pattern 182 may include, for example, silicon oxide, silicon nitride, or silicon oxynitride.

Additionally, the second temperature sensor 170 is configured to surround the heat transfer member 180 around the outlet 107.

Meanwhile, an insulating member 190 (see FIG. 1) may be additionally provided to surround the second temperature sensor 170.

The insulating member 190 can suppress error occurrence in temperature data measured by the second temperature sensor 170 due to heat retained by the substrate 100 by thermally isolating the second temperature sensor 170 from the substrate.

FIG. 4 is a plan view illustrating a gas sensing element according to an embodiment of the present invention.

Referring to FIG. 4, the inlet 106 is arranged radially in plurality around the center of the reference region. The outlet 107 is arranged radially in plurality around the center of the sensing region.

At this time, the first temperature sensor 160 may extend in a zigzag manner along the boundaries of the inlets 106, and the second temperature sensor 170 may extend in a zigzag manner along the boundaries of the outlets 107.

As multiple inlets 106 and outlets 107 are formed in the substrate, the area capable of sensing gas temperature can be expanded. Therefore, the gas sensing element 10 can secure superior sensitivity.

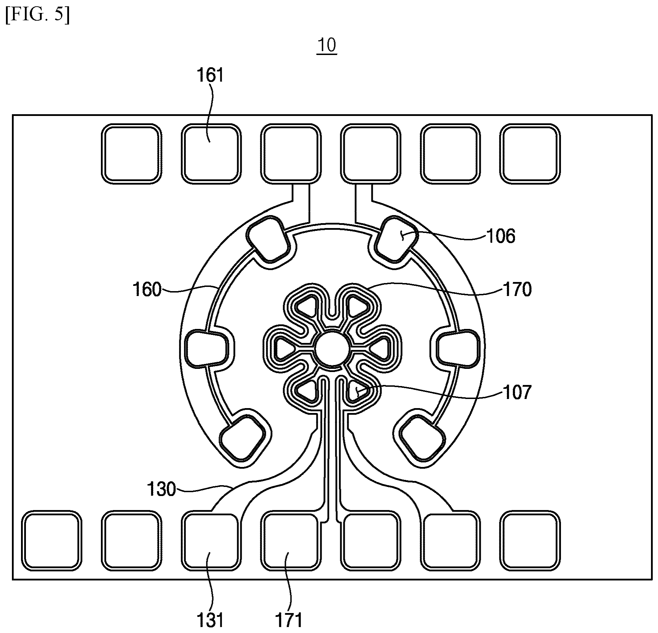

FIG. 5 is a plan view illustrating a gas sensing element according to an embodiment of the present invention.

Referring to FIG. 5, the reference region is formed to surround the sensing region, the inlet 106 is arranged radially in plurality around the center of the sensing region, and the outlet 107 is arranged radially in plurality around the center of the sensing region.

At this time, the first temperature sensor 160 may extend along the inlets 106, and the second temperature sensor 170 may extend in a zigzag manner along the boundaries of the outlets 107.

### INDUSTRIAL APPLICABILITY

The gas sensing element can be used in various applications in the technical field of detecting hydrogen gas using thermal conductivity methods.

While the above has been described with reference to preferred embodiments of the present invention, those skilled in the art will understand that various modifications and changes can be made to the present invention within the scope not departing from the spirit and scope of the present invention described in the following claims.

## Claims

1. A gas sensing element comprising:
a substrate having a reference region and a sensing region spaced apart from each other, the substrate including an inlet located in the reference region through which gas enters an interior of the substrate, an outlet located in the sensing region through which gas is discharged from the interior of the substrate, and a connecting channel formed inside that connects the inlet and the outlet;
a heater disposed adjacent to the outlet and configured to heat a second gas existing around the outlet to generate upward airflow, thereby naturally convecting a first gas existing around the inlet through the inlet, the connecting channel, and the outlet; and
first and second temperature sensors disposed around each of the inlet and outlet and configured to sense temperatures of the first and second gases, respectivley.

2. The gas sensing element of claim 1, wherein each of the first or second temperature sensors is configured to surround the inlet or outlet.

3. The gas sensing element of claim 1, wherein the heater is formed of at least one metal selected from Pt, Pt/Ti, Pt/Ta, Ir, Mo, and W.

4. The gas sensing element of claim 1, further comprising a heat transfer member configured to surround the inlet or outlet.

5. The gas sensing element of claim 4, wherein the heat transfer member comprises: a metal ring surrounding the inlet or outlet; and an insulating layer pattern embedded within the metal ring.

6. The gas sensing element of claim 4, wherein the second temperature sensor is configured to surround the heat transfer member around the outlet, and further comprises an insulating member to surround the second temperature sensor.

7. The gas sensing element of claim 1, wherein the inlet is arranged radially in plurality around the center of the reference region, and the outlet is arranged radially in plurality around the center of the sensing region.

8. The gas sensing element of claim 7, wherein the first temperature sensor extends in a zigzag manner along boundaries of the inlets, and the second temperature sensor extends in a zigzag manner along boundaries of the outlets.

9. The gas sensing element of claim 1, wherein the reference region is formed to surround the sensing region, the inlet is arranged radially in plurality around the center of the sensing region, and the outlet is arranged radially in plurality around the center of the sensing region.

10. The gas sensing element of claim 9, wherein the first temperature sensor extends along the inlets, and the second temperature sensor extends in a zigzag manner along boundaries of the outlets.
